# EUROPEAN PATENT SPECIFICATION

(11) **EP 2 800 735 B1**
(45) Date of publication and mention of the grant of the patent: **30.03.2016**
(21) Application number: 13703144.9
(22) Date of filing: 04.01.2013
(51) Int. Cl.: C07C 221/00, C07C 213/00, C07C 213/06, C07C 213/08, C07C 217/62, C07C 215/54, C07C 219/28

(54) **PROCESS FOR THE PREPARATION OF FESOTERODINE**
VERFAHREN ZUR HERSTELLUNG VON FESOTERODIN
PROCÉDÉ DE PRÉPARATION DE FÉSOTÉRODINE

(30) Priority: 07.01.2012 IN MM00622012
(43) Date of publication of application: 12.11.2014
(73) Proprietor: Alembic Pharmaceuticals Limited, Vadodara 390003 (IN); Jayaraman, Venkat Raman, Vadodara 390003 (IN); Patel, Samir, Vadodara 390003 (IN); Thakor, Indrajit, Vadodara 390003 (IN); Parekh, Viral Maheshbhai, Vadodara 390003 (IN); Ladani, Mahesh, Vadodara 390003 (IN); Patil, Chetan, Vadodara 390003 (IN); Patel, Ronak, Vadodara 390003 (IN); Raval, Prashant, Vadodara 390003 (IN)
(72) Inventor: JAYARAMAN, Venkat, Raman, Vadodara 390003 (IN); PATEL, Samir, Vadodara 390003 (IN); THAKOR, Indrajit, Vadodara 390003 (IN); PAREKH, Viral, Maheshbhai, Vadodara 390003 (IN); LADANI, Mahesh, Vadodara 390003 (IN); PATIL, Chetan, Vadodara 390003 (IN); PATEL, Ronak, Vadodara 390003 (IN); RAVAL, Prashant, Vadodara 390003 (IN)
(74) Representative: Watson, Robert James
(86) International application number: PCT/IB2013/050089
(87) International publication number: WO 2013/035084

(56) References cited:
- WO-A2-2005/012227

## Description

### FIELD OF THE INVENTION

The present invention relates to an improved, commercially viable and industrially advantageous process for the preparation of Fesoterodine or a pharmaceutically acceptable salt thereof in high yield and purity. More specifically the present invention relates to an improved and industrially advantageous oxidation method to convert O-benzyl tolterodine to Fesoterodine and salts thereof.

### BACKGROUND OF THE INVENTION

Fesoterodine is [2-[(1*R*)-3-(Di(propan-2-yl)amino)-1-phenylpropyl]-4-(hydroxymethyl) phenyl] 2-methylpropanoate and represented by formula (I).

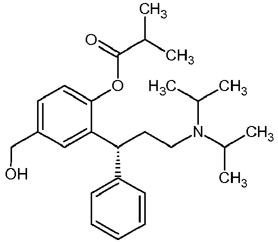

The product is marketed in the form of Fumarate salt. The current pharmaceutical product containing this drug is being sold by Pfizer using the trade name Toviaz, in the form of extended release oral tablets. Fesoterodine is cholinergic antagonist and muscarinic antagonist. Fesoterodine is rapidly de-esterified to its active metabolite, (R)-2-(3-diisopropylamino-1-phenylpropyl)-4-hydroxymethyl-phenol, or 5-hydroxy methyl tolterodine, which is a muscarinic receptor antagonist. Fesoterodine is used as Urinary Incontinence Products. It is used to treat overactive bladder.

Few processes for the synthesis of 3, 3-diphenylpropylamine derivatives have been described in the literature. Tolterodine and other 3, 3-diphenyl propylamine analogs were first described in US patent 5,382,600. US patent 6,713,464 disclosed a variety of 3,3-diphenylpropylamine derivatives, processes for their preparation, pharmaceutical compositions in which they are present and method of use thereof.

WO2005012227 describes process for preparation of Fesoterodine from Tolterodine. However, this process involves preparation of resolved Tolterodine and then its benzylation, increase the number of steps such as debenzylation to prepare tolterodine, resolution of tolterodine and then further benzylation to convert in R (+) benzyl tolterodine which further gets converted to Fesoterodine. Further this process involves oxidation of methyl group to aldehyde group using copper sulphate and sodium persulphate. However, during this conversion many impurities develop, reaction is very slow and unreacted material remains as such which reduces the overall yield and purity. Further, this process requires excess of metal persulphate which increase overall cost.

WO2012137047 describes process for preparation of Fesoterodine. In this process oxidation of O-benzyl tolterodine is carried out using ceric ammonium nitrate. The relative higher cost of ceric ammonium nitrate and the toxic effluent ingredients demand safer alternatives for this oxidation reaction.

Thus, a need still remains for an improved and commercially viable process of preparing pure Fesoterodine or a pharmaceutically acceptable salt thereof that will solve the aforesaid problems associated with process described in the prior art and will be suitable for large-scale preparation.

To address this need, a process has been developed using readily available oxidizing agent which is also environment friendly. In the present process use of metal persulphate in presence of coreductant achieves the intended transformation described below. This process gives assurance about the ease of scale up in commercial quantity.

### SUMMARY OF THE INVENTION

The present inventors have focused on the problems associated with the prior art process and has developed an improved process for the preparation of Fesoterodine.

Therefore, in one aspect, the present invention provides a process of oxidizing compound A in to compound B in presence of metal persulphate, organic or inorganic coreductant and optionally inorganic acid, 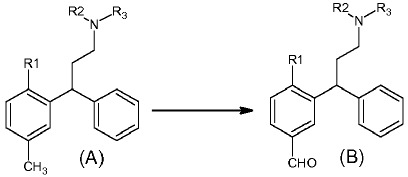
wherein, R1 is hydrogen, hydroxy, alkyl, alkoxy, hydroxyalkyl, trifluoromethyl, disubstituted amino, nitro, alkylcarbonylamino, alkylcarbonyloxy, halogen,
R2 and R3 represent C1-C6 alkyl groups, which may be the same or different and which together contain at least three carbon atoms, or R2 and R3 may form a ring together with the amine nitrogen.

In another aspect, present invention provides a process of oxidizing compound A in to compound B in presence of metal persulphate, organic or inorganic coreductant and optionally inorganic acid, 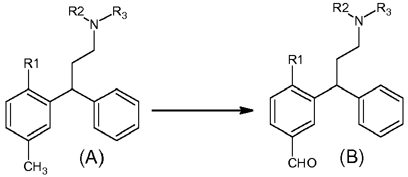
wherein, R1 is hydrogen, hydroxy, alkyl, alkoxy, hydroxyalkyl, trifluoromethyl, disubstituted amino, nitro, alkylcarbonylamino, alkylcarbonyloxy, halogen,
R2 and R3 represent C1-C6 alkyl groups, which may be the same or different and which together contain at least three carbon atoms, or R2 and R3 may form a ring together with the amine nitrogen.

In another aspect, metal persulphate is selected from the group of sodium persulphate, ammonium persulphate or potassium persulphate more preferably sodium persulphate.

In another aspect the coreductant is selected from dialkylsulphides, diaryl sulphides, alkyl aryl sulphides, dialkylsulfoxides, diarylsulfoxides, alkylarylsulfoxides, mono or poly substituted amines, mono or poly substituted phosphines; more preferably, the coreductant is selected from dimethyl sulphoxide or dimethyl sulphide.

In another aspect, the inorganic acid is selected from the group of sulphuric acid, hydrochloric acid and nitric acid.

In another aspect, present invention provides a process of oxidizing compound A in to compound B in presence of sodium persulphate, dimethyl sulphoxide and optionally with sulphuric acid. 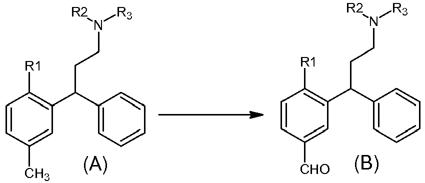
wherein, R1 is hydrogen, hydroxy or substituted or unsubstituted alkyl, aryl, aryloxy, alkoxy, hydroxyalkyl, trifluoromethyl, disubstituted amino, nitro, alkylcarbonylamino, alkylcarbonyloxy, halogen, arylalkyloxy, substituted silyloxy,
R2 and R3 represent C1-C6 alkyl groups, which may be the same or different and which together contain at least three carbon atoms, or R2 and R3 may form a ring together with the amine nitrogen.

In another aspect, present invention provides a process for preparing Fesoterodine or its enantiomer, or a salt thereof, comprising a step of oxidizing a compound of formula II or its enantiomer in to compound of formula III or its enantiomer in presence of sodium persulphate, dimethyl sulphoxide and optionally with sulphuric acid. 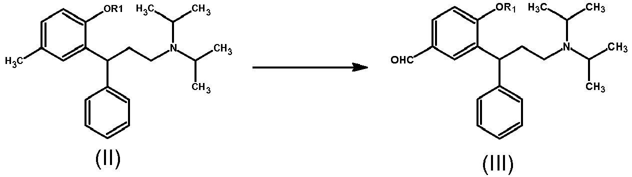 Where R1 is any hydroxyl protecting group,

In another aspect, present invention provides a process for preparing Fesoterodine or its enantiomer, or a salt thereof, comprising a step of oxidizing a compound of formula II or its enantiomer in to compound of formula III or its enantiomer using metal sulphate, metal acetate and metal persulphate in presence of inorganic acid and coreductant. 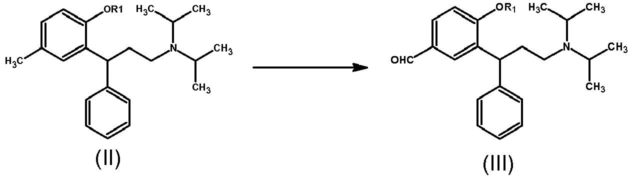 Where R1 is any hydroxyl protecting group,

In another aspect, metal sulphate is selected from the group of copper sulphate, ferrous sulphate, zinc sulphate, magnesium sulphate, manganese sulphate, more preferably ferrous sulphate.

In another aspect, metal acetate is selected from the group of sodium acetate, potassium acetate, magnesium acetate, calcium acetate, nickel acetate, copper acetate, zinc acetate, iron acetate, more preferably copper acetate.

In another aspect, present invention provides a metal persulphate is selected from the group of sodium persulphate, ammonium persulphate or potassium persulphate more preferably sodium persulphate.

In another aspect, an inorganic acid is selected from the group of sulphuric acid, hydrochloric acid, hydrobromic acid or hydroiodic acid.

In another aspect, present invention provides coreductant is selected from dimethyl sulphoxide or dimethyl sulphide.

In another aspect, present invention provides a process for preparing Fesoterodine or its enantiomer, or a salt thereof, comprising a step of oxidizing compound of formula IV to convert in compound of formula V using sodium persulphate, copper sulphate, copper acetate and sulphuric acid in presence of dimethyl sulphoxide.

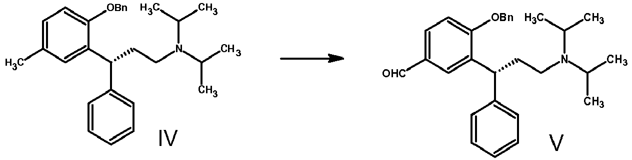

In another aspect, coreductant is selected from dimethyl sulphoxide or dimethyl sulphide.

The present inventors have found that employing process of the present invention for the preparation of Fesoterodine, overcomes the drawbacks of the prior art and may be prepared and subsequently converted to Fesoterodine in high yield and purity.

### DETAILED DESCRIPTION OF THE INVENTION:

The present invention provides a process for preparation of Fesoterodine or a pharmaceutically acceptable salt there of, which comprises:
The process for preparation of Fesoterodine Fumarate is shown in the scheme I.

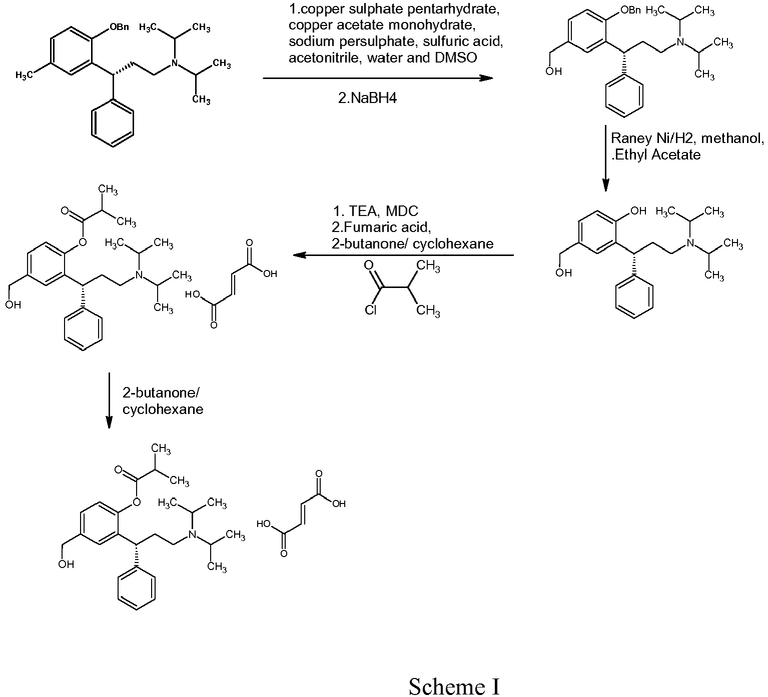

In one embodiment of the present invention provides a process for preparing Fesoterodine or its enantiomer, or a salt thereof, comprising a step of oxidizing a compound of formula II or its enantiomer in to compound of formula III or its enantiomer using metal sulphate, metal acetate and metal persulphate in presence of inorganic acid and organic solvents optionally with water. 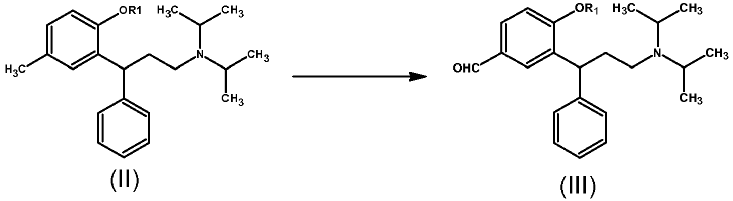 Where R1 is any hydroxyl protecting group,

Metal sulphate is selected from the group of copper sulphate, ferrous sulphate, zinc sulphate, magnesium sulphate, manganese sulphate, more preferably ferrous sulphate. coreductant is selected from dialkylsulphides, diaryl sulphides, alkyl aryl sulphides, dialkylsulfoxides, diarylsulfoxides, alkylarylsulfoxides, mono or poly substituted amines, mono or poly substituted phosphines; more preferably, the coreductant is selected from dimethyl sulphoxide or dimethyl sulphide.

Metal acetate is selected from the group of sodium acetate, potassium acetate, magnesium acetate, calcium acetate. Magnesium acetate, nickel acetate, copper acetate, zinc acetate, iron acetate, more preferably copper acetate. Metal Persulphate is selected from the group of sodium persulphate, Sodium peroxomonosulfate, Sodium peroxodisulfate, ammonium persulphate or potassium persulphate more preferably sodium persulphate. Organic solvents are selected from the group of dimethyl formamide, chloroform, acetonitrile, tetrahydrofuran, toluene, dimethyl acetamide or mixtures thereof. Inorganic acid is selected from the group of sulphuric acid, hydrochloric acid, hydrobromic acid or hydroiodic acid.

Wherein hydroxyl protecting group are selected from the group of Acetyl, Benzoyl, substituted Benzoyl, Benzyl, substituted benzyl, Dimethoxy trityl, Methoxy trityl, and the like, more preferably Benzyl.

The method of the invention makes use of persulphate, which is a stable compound which is not very toxic and has a good oxidizing power.

Therefore, in one embodiment the present invention provides a process of oxidizing compound A in to compound B in presence of metal persulphate, organic or inorganic coreductant and optionally inorganic acid, 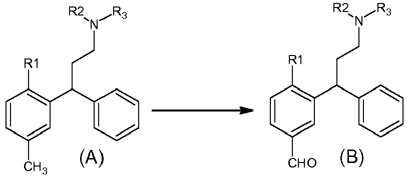
wherein, R1 is hydrogen, hydroxy or substituted or unsubstituted alkyl, aryl, aryloxy, alkoxy, hydroxyalkyl, trifluoromethyl, disubstituted amino, nitro, alkylcarbonylamino, alkylcarbonyloxy, halogen, arylalkyloxy, substituted silyloxy,
R2 and R3 represent C1-C6 alkyl groups, which may be the same or different and which together contain at least three carbon atoms, or R2 and R3 may form a ring together with the amine nitrogen.

More specifically present invention provides a process as shown in scheme-II

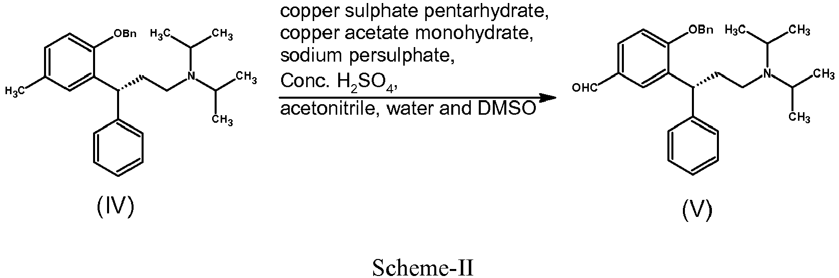

The process of the present invention has following advantages:
1. Less impurity formation.
2. Product obtained is better in terms of yield and purity, compared to other processes.
3. Generates effluent which can be handled easily.

The present invention further illustrated in detail by the below examples which are however not limit to the scope of the invention.

### Examples

### Example-1

### Preparation of R (+)-[4-benzyloxy-3-(3-diisopropylamino-1-phenyl-propyl)-phenyl]-methanol:

Charged R-(+)-N, N-diisopropyl-3-(2-benzyloxy-5-methylphenyl)-3-phenyl propane amine (51.0 g), Acetonitrile (500 ml), Process water (400 ml) and DMSO (100ml) into the reaction mass and stirred the reaction mass at 30±35°C. Slowly added conc. sulphuric acid (2.0gm) and stirred the reaction mass at 30±35°C. Charged Copper acetate monohydrate (4.13 g), Ferrous sulfate heptahyrate (13.86 g) and Sodium persulfate (59.36 g) at 30±5°C. The contents were stirred at 80°C for 1 hr followed by further addition of Copper acetate monohydrate (1.02 g), Ferrous sulfate heptahyrate (5.02 g) and Sodium persulphate (17.79 g) at 60°C. Maintained at 80°C till completion of the reaction. Added 10% aqueous solution of Sodium hydroxide (275 ml) to adjust pH 6.0 to 7.0 between 0 to 5°C followed by lot wise addition of Sodium Borohydride (14.18 g) at 5±3°C. After completion of the reaction Conc. Hydrochloric acid was added and stirred the reaction mass for 90-120 min at 35±3°C. The product was extracted in Dichloromethane (200 ml) and distilled out Dichloromethane completely to give 42.3 g R-(+)-[4-benzyloxy-3-(3-diisopropylamino-1-phenyl-propyl)-phenyl]-methanol. Yield 78.5%.

### Example-2

### Preparation of R (+)-2-(3-diisopropylamino-1-phenylpropyl)-hydroxymethyl phenol.

R-(+)-[4-benzyloxy-3-(3-diisopropylamino-1-phenyl-propyl)-phenyl]-methanol (65.0 g), Raney Nickel (40 g) and Methanol (100 ml) were taken in to a hydrogenator and Maintained 4-5 Kg/Cm² pressure of Hydrogen for 180-240 min. The mixture was then filtered and the solvent was removed by vacuum at below 50°C. The residue was crystallized in ethyl acetate (60 ml) to give 37.0 g R-(+)-[4-hydroxy-3-(3-diisopropylamino-1-phenyl-propyl)-phenyl]-methanol. Yield 72.5%.

### Example-3

### Preparation of R-(+)-2-(3-Diisopropylamino-1-phenylpropyl)-4-hydroxy methyl - phenyl isobutyrate ester hydrogen fumarate.

A solution of R-(+)-2-(3-diisopropylamino-1-phenylpropyl)-4-hydroxymethylphenol (100.0 g) and triethylamine (30.0 g) in 1200ml dichloromethane has a solution of isobutyryl chloride (34.0 g) in 500 ml dichloromethane added under agitation and cooling. Following addition agitation takes place for a further 15 minutes at 0°C, then for 30 minutes at room temperature. After completion of the reaction, reaction mass was washed with water (250 ml) and 5% aqueous sodium hydrogen carbonate solution. The organic phase is separated and concentrated on the rotary evaporator until dry. Obtained ester was dissolved in 2-butanone (300 ml) and then added fumaric acid (34.0g). Complete dissolution of the acid on slight heating. Cyclohexane (75 ml) was slowly added under agitation until the onset of turbidity. The reaction mass was stirred for 6-7hrs at room temperature and then at 3±3°C for 60-90 min. The reaction mass was filtered and washed with cold (150 ml) Methylethylketone: Cyclohexane mixture (75:25) at 3±3°C. The wet cake was dried at 30±3°C under vacuum. Yield 71%.

### Example-4

### Purification of Fesoterodine Fumarate

Methylethylketone (200 ml) and crude Fesoterodine Fumarate(100.0g) was charged in to RB Flask and warm the reaction mass at 43±2°C to get clear solution. Cyclohexane (62.5 ml) was added to the reaction mass and reaction mass was stirred for 720-840 min at 25±2°C. The reaction mass was cooled to 3±3°C for 60-90 min and then filtered it. The reaction mass was washed with cold (150 ml) Methylethylketone: Cyclohexane mixture (75:25) at 3±3°Cand then with Cyclohexane (200 ml) at 25±3°C. The wet cake was dried at 30±3°C under vacuum. Yield 90%.

## Claims

1. A process of oxidizing compound A in to compound B in presence of metal persulphate, organic or inorganic coreductant and optionally with inorganic acid, 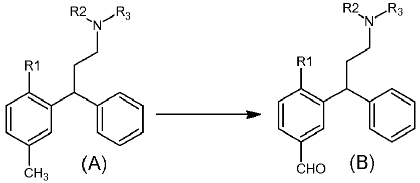
wherein, R1 is hydrogen, hydroxy or substituted or unsubstituted alkyl, aryl, aryloxy, alkoxy, hydroxyalkyl, trifluoromethyl, disubstituted amino, nitro, alkylcarbonylamino, alkylcarbonyloxy, halogen, arylalkyloxy, substituted silyloxy,
R2 and R3 represent C1-C6 alkyl groups, which may be the same or different and which together contain at least three carbon atoms, or R2 and R3 may form a ring together with the amine nitrogen.

2. A process according to claim 1, wherein metal persulphate is selected from the group of sodium persulphate, ammonium persulphate or potassium persulphate.

3. A process according to claim 1, wherein said coreductant is selected from dialkylsulphides, diaryl sulphides, alkyl aryl sulphides, dialkylsulfoxides, diarylsulfoxides, alkylarylsulfoxides, mono or poly substituted amines, mono or poly substituted phosphines.

4. A process according to claim 3, wherein said coreductant is selected from dimethyl sulphoxide or dimethyl sulphide.

5. The process according to claim 1, wherein inorganic acid is selected from the group of sulphuric acid, hydrochloric acid, hydrobromic acid or hydroiodic acid.

6. A process according to claim 1, wherein the metal persulphate is sodium persulphate, the coreductant is dimethyl sulphoxide and the inorganic acid is sulphuric acid.

7. A process according to claim 6, wherein R1 is ORA, R2 and R3 are -CH(CH₃)_{2,} where RA is selected from hydrogen, alkyl, substituted or unsubstituted aryl, arylalkyl, substituted silyl,

8. A process according to claim 1, wherein R1 is ORA, R2 and R3 are -CH(CH₃)_{2,} additionally in the presence of metal sulphate and metal acetate and in presence of inorganic acid, where RA is any hydroxyl protecting group.

9. The process according to claim 8, wherein metal sulphate is selected from the group of copper sulphate, ferrous sulphate, zinc sulphate, magnesium sulphate, manganese sulphate, more preferably ferrous sulphate.

10. The process according to claim 8, wherein metal acetate is selected from the group of sodium acetate, potassium acetate, magnesium acetate, calcium acetate, magnesium acetate, nickel acetate, copper acetate, zinc acetate, iron acetate, more preferably copper acetate.

11. The process according to claim 8, wherein metal persulphate is selected from the group of sodium persulphate, ammonium persulphate or potassium persulphate more preferably sodium persulphate.

12. The process according to claim 8, wherein inorganic acid is selected from the group of sulphuric acid, hydrochloric acid and nitric acid.

13. A process according to claim 8, wherein said coreductant is selected from dimethyl sulphoxide or dimethyl sulphide.

14. A process according to claim 8 wherein, the metal persulphate is sodium persulphate, the metal sulphate is copper sulphate, the metal acetate is copper acetate, the inorganic acid is sulphuric acid, the coreductant is dimethyl sulphoxide and the hydroxyl protecting group is benzyl.

## Patentansprüche

1. Verfahren zum Oxidieren von Verbindung A zu Verbindung B in Gegenwart von Metallpersulfat, organischem oder anorganischem Coreduktionsmittel und gegebenenfalls mit anorganischer Säure, 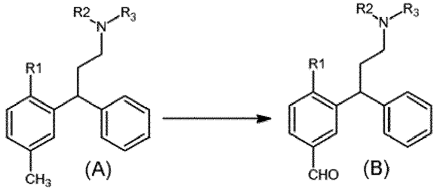
worin R1 Wasserstoff, Hydroxy oder substituiertes oder unsubstituiertes Alkyl, Aryl, Aryloxy, Alkoxy, Hydroxyalkyl, Trifluoromethyl, disubstituiertes Amino, Nitro, Alkyl-carbonylamino, Alkylcarbonyloxy, Halogen, Arylalkyloxy, substituiertes Silyloxy ist,
R2 und R3 für C₁-C₆-Alkylgruppen stehen, die gleich oder unterschiedlich sein können und die zusammen zumindest drei Kohlenstoffatome enthalten, oder gegebenenfalls R2 und R3 zusammen mit dem Aminostickstoff einen Ring bilden.

2. Verfahren nach Anspruch 1, worin das Metallpersulfat aus der aus Natriumpersulfat, Ammoniumpersulfat und Kaliumpersulfat bestehenden Gruppe ausgewählt wird.

3. Verfahren nach Anspruch 1, worin das Coreduktionsmittel aus Dialkylsulfiden, Diarylsulfiden, Alkylarylsulfiden, Dialkylsulfoxiden, Diarylsulfoxiden, Alkylarylsulfoxiden, ein- oder mehrfaach substituierten Aminen, ein- oder mehrfach substituierten Phosphinen ausgewählt wird.

4. Verfahren nach Anspruch 3, worin das Coreduktionsmittel aus Dimethylsulfoxid und Dimethylsulfid ausgewählt wird.

5. Verfahren nach Anspruch 1, worin die anorganische Säure aus der aus Schwefelsäure, Salzsäure, Bromwasserstoffsäure und lodwasserstoffsäure bestehenden Gruppe ausgewählt wird.

6. Verfahren nach Anspruch 1, worin das Metallpersulfat Natriumpersulfat ist, das Coreduktionsmittel Dimethylsulfoxid ist und die anorganische Säure Schwefelsäure ist.

7. Verfahren nach Anspruch 6, worin R1 ORA ist und R2 und R3 -CH(CH₃)₂ sind, wobei RA aus Wasserstoff, Alkyl, substituiertem oder unsubstituiertem Aryl, Arylalkyl und substituiertem Silyl ausgewählt wird.

8. Verfahren nach Anspruch 1, worin R1 ORA ist und R2 und R3 -CH(CH₃)₂ sind, zusätzlich dazu in Gegenwart von Metallsulfat und Metallacetat und in Gegenwart von anorganischer Säure, wobei RA eine beliebige Hydroxylschutzgruppe ist.

9. Verfahren nach Anspruch 8, worin das Metallsulfat aus der aus Kupfersulfat, Eisensulfat, Zinksulfat, Magnesiumsulfat, Mangansulfat bestehenden Gruppe ausgewählt wird und noch bevorzugter Eisensulfat ist.

10. Verfahren nach Anspruch 8, worin das Metallacetat aus der aus Natriumacetat, Kaliumacetat, Magnesiumacetat, Calciumacetat, Magnesiumacetat, Nickelacetat, Kupferacetat, Zinkacetat und Eisenacetat bestehenden Gruppe ausgewählt wird und noch bevorzugter Kupferacetat ist.

11. Verfahren nach Anspruch 8, worin das Metallpersulfat aus der aus Natriumpersulfat, Ammoniumpersulfat und Kaliumpersulfat bestehenden Gruppe ausgewählt wird und noch bevorzugter Natriumpersulfat ist.

12. Verfahren nach Anspruch 8, worin die anorganische Säure aus der aus Schwefelsäure, Salzsäure und Salpetersäure bestehenden Gruppe ausgewählt wird.

13. Verfahren nach Anspruch 8, worin das Coreduktionsmittel aus Dimethylsulfoxid und Dimethylsulfid ausgewählt wird.

14. Verfahren nach Anspruch 8, worin das Metallpersulfat Natriumpersulfat ist, das Metallsulfat Kupfersulfat ist, das Metallacetat Kupferacetat ist, die anorganische Säure Schwefelsäure ist, das Coreduktionsmittel Dimethylsulfoxid ist und die Hydroxylschutzgruppe Benzyl ist.

## Revendications

1. Procédé d'oxydation du composé A en le composé B en présence de persulfate de métal, d'un coréducteur organique ou inorganique et facultativement avec un acide inorganique, 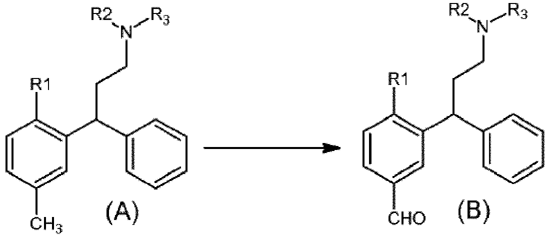
formules dans lesquelles R1 représente un atome d'hydrogène, un groupe hydroxy ou un groupe alkyle substitué ou non substitué, aryle, aryloxy, alcoxy, hydroxyalkyle, trifluorométhyle, amino disubstitué, nitro, alkylcarbonylamino, alkylcarbonyloxy, un atome d'halogène, un groupe arylalkyloxy ou un groupe silyloxy substitué,
R2 et R3 représentent des groupes alkyles en C1 à C6, qui peuvent être identiques ou différents et qui contiennent ensemble au moins trois atomes de carbone, ou R2 et R3 peuvent former un noyau conjointement avec l'azote de l'amine.

2. Procédé selon la revendication 1, dans lequel le persulfate de métal est choisi dans le groupe constitué du persulfate de sodium, du persulfate d'ammonium ou du persulfate de potassium.

3. Procédé selon la revendication 1, dans lequel ledit coréducteur est choisi parmi les dialkylsulfures, les diarylsulfures, les alkylarylsulfures, les dialkylsulfoxydes, les diarylsulfoxydes, les alkylarylsulfoxydes, les amines mono ou poly substituées, les phosphines mono ou poly substituées.

4. Procédé selon la revendication 3, dans lequel ledit coréducteur est choisi parmi le sulfoxyde de diméthyle ou le sulfure de diméthyle.

5. Procédé selon la revendication 1, dans lequel l'acide inorganique est choisi dans le groupe constitué de l'acide sulfurique, de l'acide chlorhydrique, de l'acide bromhydrique ou de l'acide iodhydrique.

6. Procédé selon la revendication 1, dans lequel le persulfate de métal est du persulfate de sodium, le coréducteur est du sulfoxyde de diméthyle et l'acide inorganique est de l'acide sulfurique.

7. Procédé selon la revendication 6, dans lequel R1 représente un groupe ORA, R2 et R3 représentent un groupe -CH(CH₃)₂, où RA est choisi parmi un atome d'hydrogène ou un groupe alkyle, aryle substitué ou non substitué, arylalkyle, ou silyle substitué.

8. Procédé selon la revendication 1, dans lequel R1 représente un groupe ORA, R2 et R3 représentent un groupe -CH(CH₃)₂, en outre en présence de sulfate de métal et d'acétate de métal et en présence d'acide inorganique, où RA représente tout groupe protecteur hydroxyle.

9. Procédé selon la revendication 8, dans lequel le sulfate de métal est choisi dans le groupe constitué du sulfate de cuivre, du sulfate ferreux, du sulfate de zinc, du sulfate de magnésium, du sulfate de manganèse, plus préférablement du sulfate ferreux.

10. Procédé selon la revendication 8, dans lequel l'acétate de métal est choisi dans le groupe constitué de l'acétate de sodium, de l'acétate de potassium, de l'acétate de magnésium, de l'acétate de calcium, de l'acétate de magnésium, de l'acétate de nickel, de l'acétate de cuivre, de l'acétate de zinc, de l'acétate de fer, plus préférablement de l'acétate de cuivre.

11. Procédé selon la revendication 8, dans lequel le persulfate de métal est choisi dans le groupe constitué du persulfate de sodium, du persulfate d'ammonium ou du persulfate de potassium, plus préférablement du persulfate de sodium.

12. Procédé selon la revendication 8, dans lequel l'acide inorganique est choisi dans le groupe constitué de l'acide sulfurique, de l'acide chlorhydrique et de l'acide nitrique.

13. Procédé selon la revendication 8, dans lequel ledit coréducteur est choisi parmi le sulfoxyde de diméthyle ou le sulfure de diméthyle.

14. Procédé selon la revendication 8 dans lequel le persulfate de métal est du persulfate de sodium, le sulfate de métal est du sulfate de cuivre, l'acétate de métal est de l'acétate de cuivre, l'acide inorganique est de l'acide sulfurique, le coréducteur est du sulfoxyde de diméthyle et le groupe protecteur hydroxyle est du benzyle.
